# EUROPÄISCHE PATENTSCHRIFT

(11) **EP 3 541 444 B1**
(45) Veröffentlichungstag und Bekanntmachung des Hinweises auf die Patenterteilung: **17.02.2021**
(21) Anmeldenummer: 17797941.6
(22) Anmeldetag: 14.11.2017
(51) Int. Cl.: A61M 1/16

(54) **HALTERUNG ZUR BEFESTIGUNG AN EINEM MEDIZINISCHEN GERÄT**
HOLDER FOR ATTACHMENT TO A MEDICAL DEVICE
SUPPORT À FIXER SUR UN APPAREIL MÉDICAL

(30) Priorität: 15.11.2016 DE 102016013623
(43) Veröffentlichungstag der Anmeldung: 25.09.2019
(73) Patentinhaber: Fresenius Medical Care Deutschland GmbH, 61352 Bad Homburg (DE)
(72) Erfinder: OFFERMANNS, Lars, 35510 Butzbach (DE)
(74) Vertreter: Herrmann, Uwe
(86) Internationale Anmeldenummer: PCT/EP2017/079193
(87) Internationale Veröffentlichungsnummer: WO 2018/091458

(56) Entgegenhaltungen:
- WO-A1-97/47336
- WO-A1-99/24088
- US-A1- 2014 008 507
- US-A1- 2016 022 894
- US-B1- 6 409 131

## Beschreibung

Die vorliegende Erfindung betrifft eine Halterung zur Befestigung an einem medizinischen Gerät, insbesondere zur Befestigung an einem Blutbehandlungsgerät.

Aus dem Stand der Technik sind verschiedene Halterungen bekannt, beispielsweise ist aus der US 2016/02289 A1 eine Halterung für einen Dialysator bekannt. Auch die Druckschrift US2014/008507 A1 offenbart eine Halterung für ein medizinisches Gerät, umfassend einen Halter und ein zweites Bauteil, an welchem der Halter angebracht ist, wobei der Halter eine Aufnahme aufweist, an welcher ein Funktionsteil lösbar einbringbar ist. Ein Blutbehandlungsgerät mit einer Halterung ist aus der WO97/47336A1 bekannt. Weiterhin offenbart die Druckschrift WO99/24088A1 eine Halterung für ein Blutreservoir. Aus der US6409131B1 ist eine Halterung bekannt, welche besonders schnell montiermar und demontierbar ist.

Häufig tritt der Fall ein, dass an medizinischen Geräten, wie beispielsweise Blutbehandlungsgeräten zusätzliche Komponenten befestigt werden müssen, wie zum Beispiel Absorber, ein Filter, ein Dialysator oder sonstige Kartuschen.

Ein Nachteil bei der Befestigung eines solchen Gegenstandes an einem Blutbehandlungsgerät besteht darin, dass jeder Gegenstand, der zusätzlich bzw. nachträglich an dem Blutbehandlungsgerät befestigt wird, die Abmessungen des Geräts vergrößert.

Dies ist insbesondere beim Einsatz eines Blutbehandlungsgerätes in intensivmedizinischen Abteilungen, in welchen nur sehr wenig Raum zur Unterbringung der medizinisch notwendigen Versorgung zur Verfügung steht, von Nachteil.

Abgesehen davon stellen zusätzlich an einem medizinischen Gerät angebrachte Kartuschen, wie beispielsweise Absorber, Filter oder Dialysatoren grundsätzlich eine Gefahr für den Benutzer bzw. für den Patienten dar, da dieser möglichweise an dem angebrachten Gegenstand hängen bleibt, sich verletzt oder das Gerät kippt, da durch den angehängten Gegenstand der Schwerpunkt des Gerätes verlagert ist.

Ein weiterer Aspekt bei der Befestigung zusätzlicher Gegenstände an einem medizinischen Gerät besteht darin, dass der Gegenstand keine wesentlichen Komponenten des Gerätes verdecken darf, vorzugsweise müssen sämtliche Maschinenkomponenten, wie beispielsweise die Tropfkammer, die Pumpe etc. stets gut sichtbar sein, das heißt nicht durch die Kartusche etc. oder deren Halterung verdeckt werden, damit sie stets einsehbar und kontrollierbar bzw. bedienbar sind.

Bei vielen medizinischen Geräten, insbesondere bei Blutbehandlungsgeräten kommt zudem ein Schlauchset zum Einsatz, das in einer bestimmten Art und Weise medizinische Flüssigkeiten bzw. Blut führt. Bei der Befestigung eines Gegenstandes an einem medizinischen Gerät ist zusätzlich darauf zu achten, dass die Schlauchführung nicht beeinflusst wird.

Der vorliegende Erfindung liegt die Aufgabe zugrunde, eine Halterung zu schaffen, die zur Befestigung an einem medizinischen Gerät und insbesondere an einem Blutbehandlungsgerät geeignet und bestimmt ist, und die eine kompakte, gefahrlose und funktionale Anordnung eines Gegenstandes an dem Blutbehandlungsgerät bzw. an den medizinischen Gerät ermöglicht.

Diese Aufgabe wird durch eine Halterung mit den Merkmalen des Anspruchs 1 gelöst.

Bei einer erfindungsgemäßen Halterung ist vorgesehen, dass die Halterung einen Befestigungsabschnitt aufweist, der ausgebildet ist, um an einer Stange des medizinischen Gerätes befestigt zu werden, dass die Halterung darüber hinaus ein Halteelement aufweist, mittels dessen ein Dialysator an der Halterung fixierbar ist und dass die Halterung ein Mittelteil aufweist, das sich zwischen dem Befestigungsabschnitt und dem Halteelement erstreckt und relativ zu dem Befestigungsabschnitt und dem Halteelement bewegbar ist.

Die erfindungsgemäße Halterung weist somit einen Befestigungsabschnitt auf, mittels dessen die Halterung zum Beispiel an einer Griffstange eines Dialysegerätes befestigt werden kann. Der Befestigungsabschnitt steht bewegbar mit einem Mittelteil in Verbindung, das seinerseits bewegbar mit einem Halteelement in Verbindung steht, mittels dessen ein Dialysator fixiert werden kann. Vorzugsweise besteht die Verbindung zwischen Mittelteil und Halteelement bzw. zwischen Mittelteil und Befestigungsabschnitt jeweils aus einem Drehgelenk, das eine Drehbewegung des Befestigungsabschnittes relativ zum Mittelteil sowie des Halteelementes relativ zum Mittelteil ermöglicht.

Grundsätzlich kommt jedes beliebige Element bzw. Verbindungselement in Betracht, das eine Relativbewegung zwischen den genannten Teilen ermöglicht, wie beispielsweise auch ein Kugelgelenk oder auch ein elastisch verformbares Element. Somit ist eine Bewegung um genau eine Achse oder auch um mehr als eine Achse denkbar und von der Erfindung umfasst.

Durch diese Ausführung einer Halterung ist es möglich, die Halterung ohne weiteres zu bewegen, bzw. so zu positionieren und deren einzelne Bestandteile so anzuordnen, dass der mittels der Halterung fixierte Gegenstand nicht störend angeordnet ist, kein Verletzungsrisiko mit sich bringt und im Übrigen auch raumsparend beispielsweise in einer Nische des Gerätes untergebracht werden kann.

Denkbar ist es, dass der Befestigungsabschnitt zumindest teilweise die Form einer Rohrhülse aufweist. Diese Rohrhülse kann in Längsrichtung zum Beispiel auf eine Griffstange aufgeschoben werden oder im Falle einer durchgehenden länglichen Ausnehmung der Befestigungsabschnitte auch senkrecht zur Längsrichtung auf eine Griffstange des Geräts aufgesteckt werden.

Denkbar ist es, dass der Befestigungsabschnitt eine Ausbuchtung aufweist, um eine unbeabsichtigtes Hin- und Her-Bewegen der Halterung auf einem Griff des medizinischen Geräts zu verhindert. Diese Ausbuchtung kann beispielsweise mit einem Gegenstück des medizinischen Griffes so formschlüssig zusammenwirken, dass die Halterung nicht entlang des Griffes bewegbar ist.

Erfindungsgemäß ist vorgesehen, dass das Mittelteil relativ zu dem Befestigungsabschnitt um eine erste Drehachse drehbar ist und relativ zu dem Halteelement um eine zweite Drehachse drehbar ist, wobei die erste Drehachse und die zweite Drehachse nicht parallel zueinander verlaufen, sondern senkrecht zueinander stehen.

Das Halteelement kann zwei relativ zueinander bewegbare Halteseiten bzw. - Backen aufweisen. Dabei ist vorzugsweise vorgesehen, dass eine der Halteseiten in Richtung zu der anderen Halteseite hin vorgespannt ist, sodass ein Gegenstand einfach eingeklemmt werden kann.

Das Halteelement kann derart ausgeführt sein, dass in diesem ein medizinischer Gegenstand, vorzugsweise ein zylindrischer Gegenstand aufnehmbar ist.

In einer denkbaren Ausgestaltung der Erfindung ist vorgesehen, dass in dem Halteelement ein Filter, ein Dialysator, ein Absorber oder eine sonstige Kartusche aufgenommen ist.

Wie bereits oben ausgeführt ist es vorteilhaft, wenn der Befestigungsabschnitt derart ausgebildet ist, dass er auf eine Griffstange eines medizinischen Gerätes aufsteckbar und/oder aufschiebbar ist. Dabei ist es besonders vorteilhaft, wenn die Fixierung des Befestigungsabschnittes auf der Griffstange durch eine Klemmverbindung erfolgt d.h. durch eine reibschlüssige Verbindung.

Weiterhin kann vorgesehen sein, dass der Befestigungsabschnitt einen umlaufend geschlossenen Bereich aufweist.

Dies bringt den Vorteil mit sich, dass der Befestigungsabschnitt und damit die gesamte Halterung nur in Längsrichtung von einer Griffstange des medizinischen Gerätes abgezogen werden kann. Ein unbeabsichtigtes Abfallen der Halterung in einer anderen Richtung ist ausgeschlossen.

Alternativ oder zusätzlich kann vorgesehen sein, dass der Befestigungsabschnitt einen Bereich aufweist, in dem zwei Abschnitte des Befestigungsabschnittes durch eine in Längsrichtung des Befestigungsabschnittes verlaufende Ausnehmung voneinander getrennt sind. Diese Ausführung bringt den Vorteil mit sich, dass diese beiden Abschnitte relativ zueinander vorgespannt sein können, sodass der Befestigungsabschnitt auf die Griffstange des medizinischen Gerätes aufgeklemmt werden kann und dadurch bereits eine gewisse Fixierung erfährt.

Die vorliegende Erfindung betrifft des Weiteren ein medizinisches Gerät, insbesondere ein Blutbehandlungsgerät, wie zum Beispiel ein Dialysegerät, an dem eine Halterung gemäß der Erfindung befestigt ist. Dabei ist vorzugsweise vorgesehen, dass das medizinische Gerät zum Beispiel an seiner Vorderseite eine Griffstange aufweist und dass die Halterung an dieser Griffstange befestigt ist.

Es wird darauf hingewiesen, dass die Begriffe "ein" oder "eine" nicht auf genau ein Element beschränkt sind, sondern auch eine Mehrzahl der fraglichen Elemente umfassen.

Weitere Einzelheiten und Vorteile werden anhand eines in der Zeichnung dargestellten Ausführungsbeispiels näher erläutert.

Es zeigen:
- Figur 1:: Eine Explosionsdarstellung der erfindungsgemäßen Halterung,
- Figur 2:: eine Seitenansicht der erfindungsgemäßen Halterung gemäß Figur 1,
- Figur 3:: eine Längsschnittansicht gemäß der Schnittlinie A-A in Figur 5
- Figur 4:: eine teilweise geschnittene Darstellung gemäß der Schnittlinie C-C in Figur 5,
- Figur 5:: eine Ansicht von unten auf die Halterung gemäß Figur 1 und
- Figur 6:: eine perspektivische Ansicht der Halterung gemäß der Erfindung im zusammengebauten Zustand.

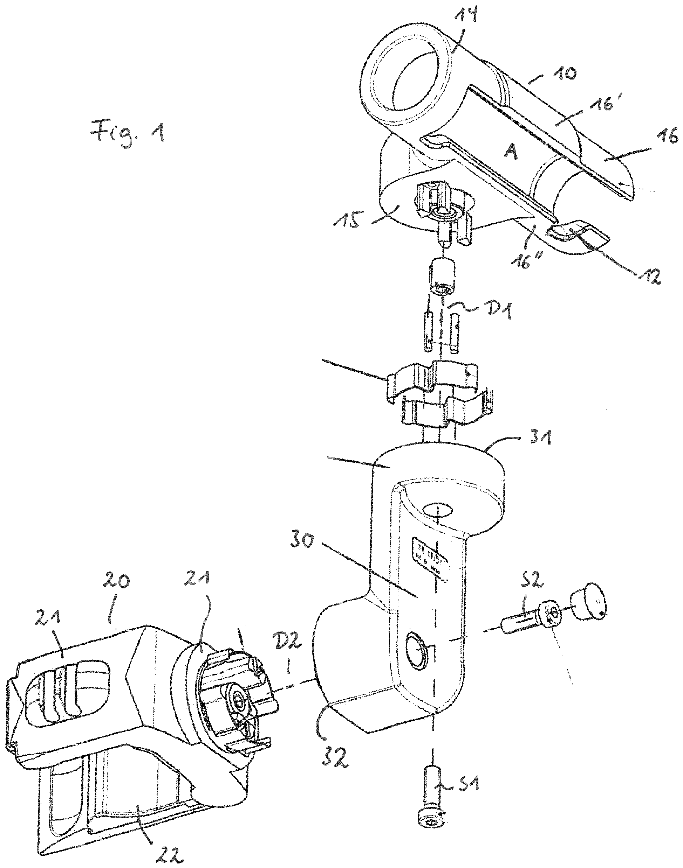

Figur 1 zeigt mit dem Bezugszeichen 10 den Befestigungsabschnitt der Halterung, der hülsenförmig bzw. rohrförmig ausgeführt ist und einen umlaufend geschlossenen Bereich 14 aufweist. An diesen Bereich schließt sich der Bereich 16 an, der eine in Längsrichtung des Befestigungsabschnitt 10 verlaufende Ausnehmung A aufweist, die durch die Beabstandung der beiden Wandungsabschnitte 16' und 16"des hülsenförmigen Befestigungsabschnitts 10 gebildet wird.

Das Bezugszeichen 12 kennzeichnet eine Ausnehmung in einer Wandung des Befestigungsabschnittes. Diese wirkt im aufgesteckten Zustand an die Griffstange eines medizinischen Geräts mit einem dort befindlichen Vorsprung zusammen, sodass die Position des Befestigungsabschnittes in Längsrichtung der Griffstange festgelegt ist.

Das Bezugszeichen D1 kennzeichnet eine erste Drehachse, um die das Mittelteil 30 relativ zu dem Befestigungsabschnitt 10 drehbar ist. Die Drehbewegung wird durch den Bolzen S1 bewirkt, der in dem Mittelteil gehalten wird sowie auch in dem Befestigungsabschnitt und die Drehachse D1 bildet.

Das Mittelteil 30 weist eine erste Fläche 31 auf, die mit einer Gegenfläche 15 des Befestigungsabschnittes 10 zusammenwirkt und die relativ zueinander drehbar sind. Senkrecht zu dieser ersten Fläche 31 verläuft die zweite Fläche 32 des Mittelteils, die mit der Gegenfläche 21 der Halterung 20 zusammenwirkt und relativ zu dieser drehbar ist.

Wie dies auf Figur 1 ersichtlich ist, ist das Mittelteil somit S-förmig ausgeführt, wobei die erste Drehachse D1 durch den oberen Abschnitt verläuft und die zweite Drehachse D2, die dazu senkrecht verläuft, durch den unteren Abschnitt. Anstatt der Bewegung um jeweils genau eine Achse ist auch eine mehrachsige Bewegung bzw. Ausführung von der Erfindung umfasst.

Die zweite Drehachse D2 wird durch den Bolzen S2 bereitgestellt, der wie auch der Bolzen S1 durch eine entsprechende Bohrung des Mittelteils 30 gesteckt wird und der mit einem entsprechenden Gegenstück bzw. einer Ausnehmung des Halteelements 20 zusammenwirkt, sodass das Halteelement relativ zu dem Mittelteil drehbar ist.

Das Halteelement 20 umfasst zwei gegenüber liegende Backen 21 und 22, eine oder beide dieser Backen können so vorgespannt sein, dass sie eine Kraft auf die andere Backe hin ausüben, sodass ein aufgenommener Gegenstand durch eine Klemmung fixiert wird.

Sämtliche der genannten Teile können aus Kunststoff oder aus einem anderen Material, wie beispielsweise Metall bestehen.

Der umlaufende Abschnitt 14 des Befestigungsabschnittes 10 bewirkt, dass der Befestigungsabschnitt 10 und damit die gesamte Halterung nicht in einer Richtung abweichend von der Längsrichtung der Griffstange bzw. des Befestigungsabschnittes 10 abgenommen werden kann, sondern nur in Längsrichtung aufgesteckt und abgezogen werden kann.

Grundsätzlich ist auch eine Ausführung denkbar, bei der der Befestigungsabschnitt 10 keinen solchen umlaufenden Abschnitt 14 aufweist, bei dem somit die Ausnehmung A sich über die gesamte Längsseite bzw. Länge erstreckt, sodass der Befestigungsabschnitt auch in eine Richtung senkrecht zur Längsrichtung der Griffstange aufgesteckt bzw. aufgeklipst werden kann.

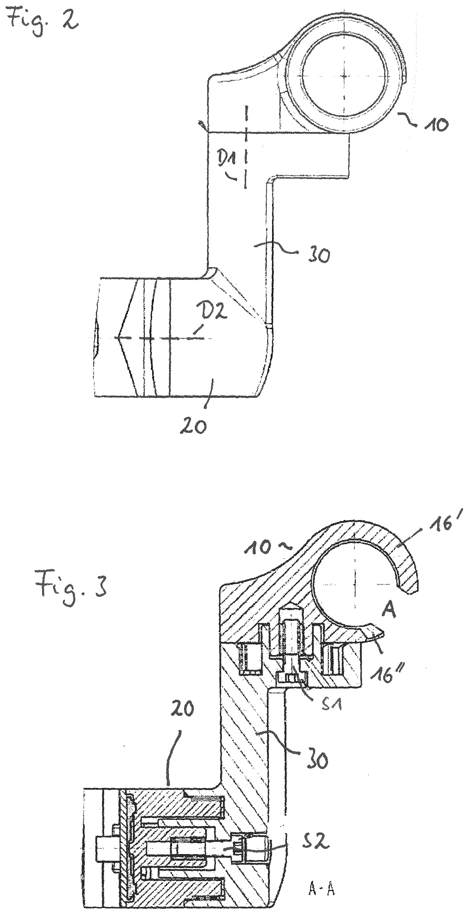

Figur 2 zeigt in einer Seitenansicht die Anordnung gemäß Figur 1 mit dem Befestigungsabschnitt 10, dem Mittelteil 30 und dem Halteelement 20. Ebenfalls angedeutet sind die Drehachsen D1 und D2, die wie aus Figur 2 ersichtlich zueinander senkrecht stehen.

Abweichend von einer derartigen senkrechten Ausgestaltung ist es in einer nicht erfindungsgemäßen Ausführungsform auch denkbar, dass die Drehachsen schräg zueinander verlaufen, jedoch nicht im rechten Winkel.

Figur 3 zeigt eine Längsschnittansicht durch die erfindungsgemäße Halterung gemäß der Schnittlinie A-A. Aus dieser Ansicht ist ersichtlich, dass auf Höhe der Schnittebene der Aufnahmebereich für die Griffstange eines medizinischen Gerätes nicht geschlossen ist, sondern eine Ausnehmung A aufweist, die von den Randbereichen der Wandungselementen 16' und 16" begrenzt wird. Wie dies weiter auf Figur 3 ersichtlich ist, ist das Mittelteil 30 mittels des Bolzens S1 drehbar relativ zu dem Befestigungsabschnitt 10 sowie auch drehbar zu dem Halteelement 20. Hierzu dient der zweite Bolzen S2.

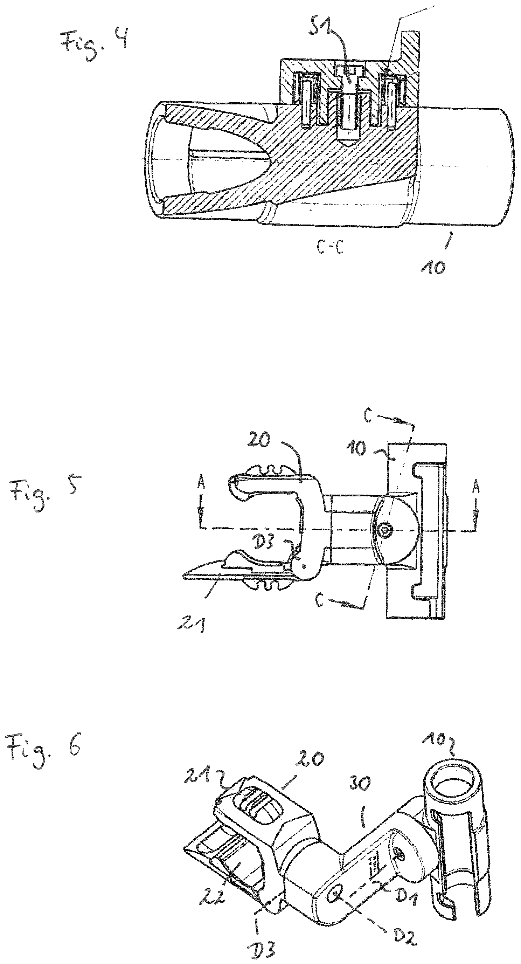

Figur 4 zeigt eine Schnittansicht der Anordnung gemäß Figur 6 entsprechend der Schnittlinie C-C und zeigt die Anordnung des ersten Bolzens S1, der die Drehachse D1 bildet.

Figur 5 zeigt die Ansicht einer Halterung von unten und verdeutlicht, dass in einem Randbereich des Befestigungsabschnitt eine umlaufende, d.h. ringförmige Ausbildung aufweist und im Übrigen eine sich über den verbleibenden Teil seiner Länge erstreckende Ausnehmung A aufweist.

Das Halteelement weist zwei Backen auf, die symmetrisch zueinander angeordnet sein können oder auch unsymmetrisch bzw. ungleich ausgeführt sein können, wie dies gemäß Figur 5 der Fall ist. Aus Figur 5 ergibt sich weiter, dass die unten dargestellte Haltebacke 22 über eine Drehachse D3 verschwenkbar ist und somit auf die andere, feststehende Haltebacke zubewegt werden kann und von dieser wegbewegt werden kann. Denkbar ist es, dass die bewegbare Haltebacke zu der anderen Haltebacke hin vorgespannt ist, sodass ohne weiteres eine Kartusche durch eine Klemmung in dem Halteelement aufgenommen werden kann. Die Haltebacken weisen in ihrem Einführbereich Abrundungen bzw. Schrägen auf, die das Einführen einer Kartusche, wie beispielsweise eines Dialysators, eines Filters oder eins Absorbers erleichtern. Nach dem Einschieben bzw. Einstecken der Kartusche wird diese selbsttätig in der Halterung fixiert, sodass keine zusätzlichen Maßnahmen, wie ein Verschrauben oder Verspannen etc. notwendig sind. Um einen sicheren Halt innerhalb des Halteelements zu gewährleisten ist es denkbar, dass das Halteelement auf seiner Innenseite rutschfeste Materialien aufweist, wie zum Beispiel eine Gummischicht.

Figur 6 zeigt schließlich in einer perspektivischen Ansicht die gesamte Halterung mit Befestigungsabschnitt 10, Mittelteil 30 und Halteelement 20 sowie eine schematische Ansicht der zueinander senkrecht verlaufenden Drehachsen D1 und D2.

Im Betrieb eines Dialysegerätes wird die Halterung mittels des Befestigungsabschnittes 10 an einer Griffstange eines Dialysegerätes befestigt, die sich beispielsweise an dessen Frontseite befindet. In das Halteelement 20 wird eine Kartusche oder ein sonstiger Gegenstand eingesteckt, der für eine Behandlung benötigt wird. Durch die zwei Gelenke, die im Ausführungsbeispiel als Drehgelenke ausgeführt sind, jedoch auch jede andere Form bzw. Gestaltung aufnehmen und annehmen können, wie beispielsweise als Kugelgelenke ausgeführt werden können oder als Scharniergelenke oder als elastische Elemente, ist es möglich, das Halteelement relativ zu der Griffstange bzw. relativ zu dem Befestigungsabschnitt in weitgehend jeder beliebigen Position anzuordnen, sodass der darin befestigte Gegenstand weder störend ist, noch Komponenten eines Blutbehandlungsgerätes wie zum Beispiel eines Dialysegerätes verdeckt. Somit ist eine sichere und funktionale Befestigung eines Gegenstandes an einem Dialysegerät oder an einem sonstigen medizinischen Gerät möglich.

## Patentansprüche

1. Halterung zur Befestigung an einem medizinischen Gerät, vorzugsweise zur Befestigung an einem Blutbehandlungsgerät, umfassend:
einen Befestigungsabschnitt (10), der dazu ausgebildet ist, um an einer Stange des medizinischen Gerätes befestigt zu werden,
ein Halteelement (20), mittels dessen ein Dialysator an dem medizinischen Gerät fixierbar ist, und
ein Mittelteil (30), das sich zwischen dem Befestigungsabschnitt (10) und dem Halteelement (20) erstreckt und relativ zu dem Befestigungsabschnitt (10) und dem Halteelement (20) bewegbar ist, wobei
das Mittelteil (30) relativ zu dem Befestigungsabschnitt (10) um eine erste Drehachse (D1) und relativ zu dem Halteelement (20) um eine zweite Drehachse (D2) drehbar ist,
**dadurch gekennzeichnet, dass**
die erste Drehachse (D1) und die zweite Drehachse (D2) senkrecht zueinander verlaufen.

2. Halterung nach Anspruch 1, **dadurch gekennzeichnet, dass** der Befestigungsabschnitt (10) die Form einer Rohrhülse aufweist.

3. Halterung nach Anspruch 1 oder 2, **dadurch gekennzeichnet, dass** der Befestigungsabschnitt (10) eine Ausbuchtung (12) aufweist, um eine unbeabsichtigte Hin- und Herbewegung der Halterung auf einem Griff des medizinischen Gerätes zu verhindern.

4. Halterung nach einem der vorhergehenden Ansprüche, **dadurch gekennzeichnet, dass** das Halteelement (20) zwei relativ zueinander bewegbare Halteseiten aufweist, wobei vorzugsweise vorgesehen ist, dass eine der Halteseiten in Richtung zu der anderen Halteseite hin vorgespannt ist.

5. Halterung nach einem der vorhergehenden Ansprüche, **dadurch gekennzeichnet, dass** in dem Halteelement (20) ein medizinischer Gegenstand in Form eines Filters, Dialysators, Adsorbers oder einer sonstigen Kartusche aufgenommen ist.

6. Halterung nach einem der vorhergehenden Ansprüche, **dadurch gekennzeichnet, dass** der Befestigungsabschnitt (10) derart ausgebildt ist, dass dieser auf eine Griffstange eines medizinischen Gerätes aufsteckbar ist und/oder auf der Griffstange durch eine Klemmverbindung fixiert wird.

7. Halterung nach einem der vorhergehenden Ansprüche, **dadurch gekennzeichnet, dass** der Befestigungsabschnitt (10) einen umlaufend geschlossenen Bereich (14) aufweist und/oder einen Bereich (16), in dem zwei Abschnitte (16', 16") des Befestigungsabschnittes (10) durch eine in Längsrichtung des Befestigungsabschnittes (10) verlaufende Ausnehmung (A) voneinander getrennt sind.

8. Medizinisches Gerät, vorzugsweise Blutbehandlungsgerät, **dadurch gekennzeichnet, dass** an dem medizinzischen Gerät eine Halterung nach einem der Ansprüche 1 bis 7 befestigt ist, wobei vorzugsweise vorgesehen ist, dass das medizinzische Gerät eine Griffstange aufweist und dass die Halterung an der Griffstange befestigt ist.

## Claims

1. A holder for attachment to a medical appliance, preferably for attachment to a blood treatment device, comprising:
an attachment portion (10) which is formed to be attached to a rod of the medical appliance,
a holding element (20) by means of which a dialyzer can be fixed to the medical appliance, and
a middle part (30) which extends between the attachment portion (10) and the holding element (20) and is movable relative to the attachment portion (10) and the holding element (20), wherein
the middle part (30) is rotatable relative to the attachment portion (10) about a first axis of rotation (D1) and relative to the holding element (20) about a second axis of rotation (D2),
**characterized in that**
the first axis of rotation (D1) and the second axis of rotation (D2) extend perpendicularly to each other.

2. The holder according to claim 1, **characterized in that** the attachment portion (10) has the shape of a tubular sleeve.

3. The holder according to claim 1 or 2, **characterized in that** the attachment portion (10) includes a bulge (12), in order to prevent an inadvertent reciprocation of the holder on a handle of the medical appliance.

4. The holder according to any of the preceding claims, **characterized in that** the holding element (20) includes two holding sides movable relative to each other, wherein it preferably is provided that one of the holding sides is pretensioned in direction toward the other holding side.

5. The holder according to any of the preceding claims, **characterized in that** in the holding element (20) a medical item in the form of a filter, dialyzer, adsorber or another cartridge is accommodated.

6. The holder according to any of the preceding claims, **characterized in that** the attachment portion (10) is formed such that the same can be plugged onto a handle bar of a medical appliance and/or is fixed on the handle bar by a clamping connection.

7. The holder according to any of the preceding claims, **characterized in that** the attachment portion (10) includes a circumferentially closed region (14) and/or a region (16) in which two portions (16', 16") of the attachment portion are separated from each other by a recess (A) extending in longitudinal direction of the attachment portion (10).

8. A medical appliance, preferably blood treatment device, **characterized in that** to the medical appliance a holder according to any of claims 1 to 7 is attached, wherein it preferably is provided that the medical appliance includes a handle bar and that the holder is attached to the handle bar.

## Revendications

1. Support à fixer sur un appareil médical, de préférence à fixer sur un dispositif de traitement du sang, comprenant :
un tronçon de fixation (10), qui est conçu pour être fixée à une tige de l'appareil médical,
un élément de retenue (20), au moyen duquel un dialyseur peut être fixé à l'appareil médical, et
une partie centrale (30), qui s'étend entre le tronçon de fixation (10) et l'élément de retenue (20) et peut être déplacée par rapport au tronçon de fixation (10) et à l'élément de retenue (20), où
la partie centrale (30) peut être tournée par rapport au tronçon de fixation (10) autour d'un premier axe de rotation (D1) et par rapport à l'élément de retenue (20) autour d'un deuxième axe de rotation (D2),
**caractérisé en ce que**
le premier axe de rotation (D1) et le deuxième axe de rotation (D2) sont perpendiculaires l'un par rapport à l'autre.

2. Support selon la revendication 1, **caractérisé en ce que** le tronçon de fixation (10) possède la forme d'un manchon tubulaire.

3. Support selon la revendication 1 ou 2, **caractérisé par** en ce que le tronçon de fixation (10) présente un renflement (12) pour empêcher tout mouvement de va-et-vient accidentel du support sur une poignée de l'appareil médical.

4. Support selon l'une quelconque des revendications précédentes, **caractérisé en ce que** l'élément de retenue (20) présente deux faces de retenue déplaçable l'une par rapport à l'autre, dans lequel il est prévue de préférence que l'une des faces de retenue soit prétendue en direction de l'autre.

5. Support selon l'une quelconque des revendications précédentes, **caractérisé en ce qu'**un objet médical sous forme de filtre, de dialyseur, d'adsorbeur ou d'une autre cartouche est reçu dans l'élément de retenue (20).

6. Support selon l'une quelconque des revendications précédentes, **caractérisé en ce que** le tronçon de fixation (10) est conçu de manière à pouvoir être enfiché sur un guidon d'un appareil médical et/ou est fixée sur le guidon par une connexion de serrage.

7. Support selon l'une quelconque des revendications précédentes, **caractérisé en ce que** le tronçon de fixation (10) présente une zone circonférentiellement fermée (14) et/ou une zone (16) dans laquelle deux sections (16', 16") du tronçon de fixation (10) sont séparées l'une de l'autre par un évidement (A) s'étendant dans la direction longitudinale du tronçon de fixation (10).

8. Appareil médical, de préférence dispositif de traitement du sang, **caractérisé en ce que** sur l'appareil médical un support selon l'une quelconque des revendications 1 à 7 est fixé, il étant prévue de préférence que l'appareil médical possède un guidon et que le support soit fixé au guidon.
